# EUROPEAN PATENT SPECIFICATION

(11) **EP 3 673 081 B1**
(45) Date of publication and mention of the grant of the patent: **24.02.2021**
(21) Application number: 18756250.9
(22) Date of filing: 23.08.2018
(51) Int. Cl.: C12Q 1/6806, C12Q 1/6855, C12Q 1/6865, C12Q 1/6869

(54) **ACCURATE AND MASSIVELY PARALLEL QUANTIFICATION OF NUCLEIC ACID**
PRÄZISE UND MASSIV PARALLELE QUANTIFIZIERUNG VON NUKLEINSÄURE
QUANTIFICATION MASSIVEMENT PARALLÈLE ET PRÉCISE D'ACIDE NUCLÉIQUE

(30) Priority: 25.08.2017 EP 17188047
(43) Date of publication of application: 01.07.2020
(73) Proprietor: Eawag, Swiss Federal Institute of Aquatic Science and Technology, 8600 Dübendorf (CH)
(72) Inventor: TAMMINEN, Manu, 20500 Turku (FI); JULIAN, Timothy, 8600 Duebendorf (CH); SPAAK, Jenny, 5020 Vedrien (BE); CADUFF, Lea, 8610 Uster (CH); SCHIFF, Hanna, 2502 Biel (CH)
(74) Representative: Aaltonen, Janne Lari Antero
(86) International application number: PCT/EP2018/072754
(87) International publication number: WO 2019/038372

(56) References cited:
- US-A1- 2005 214 825
- MICHAEL S. AKHRAS ET AL: "PathogenMip Assay: A Multiplex Pathogen Detection Assay", PLOS ONE, vol. 2, no. 2, 21 February 2007 (2007-02-21), page e223, XP055358376, DOI: 10.1371/journal.pone.0000223

### SEQUENCE LISTING

<110> Eawag, Swiss Federal Institute of Aquatic Science and Technology
<120> ACCURATE AND MASSIVELY PARALLEL QUANTIFICATION OF NUCLEIC ACID
<130> Genomill001
<140> EP17188047.9
   <141> 2017-08-25
<160> 28
<170> PatentIn version 3.5
<210> 1
   <211> 31
   <212> DNA
   <213> Artificial Sequence
<220>
   <223> Example sequence 1 of figure 1
<400> 1
   ggggcccgcc gtcgatcgga gccgttagga t 31
<210> 2
   <211> 31
   <212> DNA
   <213> Artificial Sequence
<220>
   <223> Example sequence 2 of figure 1
<400> 2
   ttaaggtgcc gtcgatcgga gccgacgtac g 31
<210> 3
   <211> 31
   <212> DNA
   <213> Artificial Sequence
<220>
   <223> Example sequence 3 of figure 1
<400> 3
   ttaaggtgcc gtcgatcgga gccgacgtac g 31
<210> 4
   <211> 31
   <212> DNA
   <213> Artificial Sequence
<220>
   <223> Example sequence 4 of figure 1
<400> 4
   tataatagag gtcgtgcagt cacgacccgg t 31
<210> 5
   <211> 31
   <212> DNA
   <213> Artificial Sequence
<220>
   <223> Example sequence 5 of figure 1
<400> 5
   accaggtgcc gtcgatcgga gccgacccgg t 31
<210> 6
   <211> 31
   <212> DNA
   <213> Artificial Sequence
<220>
   <223> Example sequence 6 of figure 1
<400> 6
   gggccgggag gtcgtgcact cacgttagga t 31
<210> 7
   <211> 31
   <212> DNA
   <213> Artificial Sequence
<220>
   <223> Example sequence 7 of figure 1
<400> 7
   tccaggtgag tcgatccgtc acgtacgtac g 31
<210> 8
   <211> 31
   <212> DNA
   <213> Artificial Sequence
<220>
   <223> Example sequence 8 of figure 1
<400> 8
   aaaattttag cgtacgtcgt acgtttagga t 31
<210> 9
   <211> 31
   <212> DNA
   <213> Artificial Sequence
<220>
   <223> Example sequence 9 of figure 1
<400> 9
   tataatagag gtcgtgcagt cacgacccgg t 31
<210> 10
   <211> 31
   <212> DNA
   <213> Artificial Sequence
<220>
   <223> Example sequence 10 of figure 1
<400> 10
   aggaccttga gtcgatccgc acgtacccgg t 31
<210> 11
   <211> 31
   <212> DNA
   <213> Artificial Sequence
<220>
   <223> Example sequence 11 of figure 1
<400> 11
   agcgaccgag gtcgtgcagt cacgacgtac g 31
<210> 12
   <211> 31
   <212> DNA
   <213> Artificial Sequence
<220>
   <223> Example sequence 12 of figure 1
<400> 12
   tataatagag gtcgtgcagt cacgacccgg t 31
<210> 13
   <211> 31
   <212> DNA
   <213> Artificial Sequence
<220>
   <223> Example sequence 13 of figure 1
<400> 13
   ggaaaaagcc gtcgatcgga gccgttagga t 31
<210> 14
   <211> 31
   <212> DNA
   <213> Artificial Sequence
<220>
   <223> Example sequence 14 of figure 1
<400> 14
   atatacagag gtcgtgcagt caccttagga t 31
<210> 15
   <211> 30
   <212> DNA
   <213> Artificial Sequence
<220>
   <223> Example sequence 15 of figure 1
<400> 15
   gagacccgac ctcgtccagt cacgacccgt 30
<210> 16
   <211> 31
   <212> DNA
   <213> Artificial Sequence
<220>
   <223> Example sequence 16 of figure 1
<400> 16
   cgcacgcgag gtcgtgcagt cacgacgtac g 31
<210> 17
   <211> 29
   <212> DNA
   <213> Artificial Sequence
<220>
   <223> Example sequence 17 of figure 1
<400> 17
   tacaagccgt cgatcggagc cgacccggt 29
<210> 18
   <211> 31
   <212> DNA
   <213> Artificial Sequence
<220>
   <223> Example sequence 18 of figure 1
<400> 18
   tataatagag gtcgtgcagt cacgacccgg t 31
<210> 19
   <211> 31
   <212> DNA
   <213> Artificial Sequence
<220>
   <223> Example sequence 19 of figure 1
<400> 19
   gggcaattag cgtacgtcgt acgtacgtac g 31
<210> 20
   <211> 31
   <212> DNA
   <213> Artificial Sequence
<220>
   <223> Example sequence 20 of figure 1
<400> 20
   tatgcgagcc gtcgatcgga gccgacgtac g 31
<210> 21
   <211> 31
   <212> DNA
   <213> Artificial Sequence
<220>
   <223> Example sequence 21 of figure 1
<400> 21
   aggaccttga gtcgatccgc acgtacccgg t 31
<210> 22
   <211> 31
   <212> DNA
   <213> Artificial Sequence
<220>
   <223> Example sequence 22 of figure 1
<400> 22
   attacaagcc gtcgatcgga gccgacccgg t 31
<210> 23
   <211> 31
   <212> DNA
   <213> Artificial Sequence
<220>
   <223> Example sequence 23 of figure 1
<400> 23
   gaagaattag cgtacgtcgt acgtttagga t 31
<210> 24
   <211> 31
   <212> DNA
   <213> Artificial Sequence
<220>
   <223> Example sequence 24 of figure 1
<400> 24
   gggcaattag cgtacgtcgt acgtacgtac g 31
<210> 25
   <211> 31
   <212> DNA
   <213> Artificial Sequence
<220>
   <223> Example sequence 25 of figure 1
<400> 25
   gggcaattag cgtacgtcgt acgtacgtac g 31
<210> 26
   <211> 31
   <212> DNA
   <213> Artificial Sequence
<220>
   <223> Example sequence 26 of figure 1
<400> 26
   gagcacttag cgtacgtcgt acgtacccgg t 31
<210> 27
   <211> 31
   <212> DNA
   <213> Artificial Sequence
<220>
   <223> Example sequence 27 of figure 1
<400> 27
   aaaggggcga gtcgatccgc acgtttagga t 31
<210> 28
   <211> 31
   <212> DNA
   <213> Artificial Sequence
<220>
   <223> Example sequence 28 of figure 1
<400> 28
   agcgcgcgcc gtcgatcgga gccgttagga t 31

## Description

### TECHNICAL FIELD

The present invention disclosure relates to a next generation DNA sequencing method and use for accurate and massively parallel quantification of one or more nucleic acid targets. More particularly, the disclosure is related to the method and a kit comprising probes for detecting and quantifying genetic targets in complex DNA pools primarily used for genetic target and variant detection in human and animal populations and environmental samples. Furthermore, the invention finds particular application in the field of detection of disease-causing genetic alterations in samples obtained from human body, including without limiting biopsies, saliva and other secretions, exhaled moisture extracts, tissue, blood plasma (liquid biopsies) or the like. The invention uses one or more target-specific nucleic acid probes per genetic target (left probe and right probe) and a bridge oligo.

### BACKGROUND

With the advancement in the technology to study the genetic variation, detection of the same in plants and animals is not cumbersome. Advancement in the technology is required, however, to provide an immensely valuable study of the nucleotide sequences for genetic alterations such as mutations and other genetic variations, in order to provide necessary information for facilitating prophylactic and therapeutic measures. Nucleotide sequencing is the process of determining the nucleotide order in the genetic target of interest. The detection of mutations in the nucleotide sequence of the genetic target of interest is carried out using nucleic acid sequencing methods. Most commonly known sequencing methods such as, but not limited to, Maxam-Gilbert and Sanger are used for the detection of nucleotide sequence. The nucleic acid sequencing technology plays a vital role in numerous fields such as molecular biology, evolutionary biology, metagenomics, medicine and forensic. Ideally, detecting and quantifying genetic targets, for instance quantifying DNA in maternal blood is the basis of non-invasive prenatal trisomy screening; quantifying rare mutations in circulating DNA is the basis of cancer liquid biopsies; quantifying antibiotic resistance genes is the basis for risk estimates of antibiotic resistance gene spreading.

Typically, cancer diagnostics involve detecting and quantifying oncologically relevant mutations in circulating DNA. Similarly, fetal abnormalities involve detecting and quantifying fetal DNA, present in small concentration in maternal blood. To detect and to accurately quantify such weak signals is currently still laborious and expensive, despite decreased sequencing costs. Various problems can be expressed more accurately such as specificity in order to detect genetic signals against a consensus background, sensitivity in order to detect weak genetic signals, accuracy for accurate quantification of the detected signals, throughput number of targeted genetic targets per assay, cost per assay, scaling to determine the assay cost scale when assaying multiple samples in parallel and turn-over to determine how long is the time from sampling to the results.

Currently, the typical quantification methods for liquid biopsies and conceptually similar assays (such as antibiotic resistance gene detection) include quantitative PCR (qPCR), array qPCR, digital PCR, multiplex ligation-dependent probe Amplification (MLPA) or quantification from next-generation DNA sequencing data. While the quantification methods are robust and well-established methods, each of the method is associated with specific problems discussed in closer detail below:
Quantitative PCR: Quantitative PCR (qPCR), is a molecular biology based technique which includes the amplification of a targeted DNA molecule during the PCR, i.e. in real-time. Real-time PCR can be used quantitatively (quantitative real-time PCR), and semi-quantitatively, i.e. above/below a certain amount of DNA molecules (semi quantitative real-time PCR).
Quantitative PCR (qPCR) is a gold standard of genetic target quantification. Currently, the laboratory cost of a qPCR reaction is approximately $2. However, counting in the considerable hands on time (labour cost) for setting up the reaction, the need for standard curves, along with replicates for each quantified target, the real cost is in fact much higher. The amount of hands-on time scales steeply with an increasing number of samples since a separate quantification experiment is required for each genetic target. An estimate of at least 40 reactions per quantified target per sample sets the unit price at approximately $80, excluding the cost of manual labor, setting a definite limit for how much of the reactions can be quantified.
Array PCR: PCR Arrays are the most reliable tools for analyzing the expression of a relevant, pathway or disease focused panel of genes. Each 96-well plate, 384-well plate, or 100-well disc PCR Array includes SYBR Green-optimized primer assays for a thoroughly researched panel of focused panel of genes. A newer iteration of the qPCR technology is array qPCR which miniaturizes the individual qPCR reactions. Array PCR brings down the cost of an individual qPCR reaction and improves the scalability of the method to multiple targets and samples. However, the method is currently limited to profiling 384 targets from 12 samples (or conversely 12 targets from 384 samples) at a cost of thousands of dollars per chip plus a large capital cost of the read-out infrastructure. Profiling thousands of samples using the aforementioned setup, therefore, remains prohibitively expensive.
Digital PCR: Digital polymerase chain reaction (digital PCR, DigitalPCR, dPCR, or dePCR) is a method to provide absolute quantification of targets through droplet-microfluidics and fluorescent detection. The methodology is relatively cost-effective (one target per sample costs around $3) but the hands on time for preparing, setting-up and running individual experiments for each target in each sample scales poorly to thousands of samples.

### Multiplex Ligation-dependent Probe Amplification (MLPA)

MLPA provides an approach to simplify the detection of multiple genetic targets in individual samples. However, MLPA provides only relative quantification of targets, and requires a separate detection experiment for each sample. More recently, a variant of MLPA introduces concepts from DNA barcoding. The concept permits a better quantitative resolution and sample multiplexing than the traditional MLPA workflow. Next generation sequencing-based approaches: Next-generation sequencing (NGS), also known as high-throughput sequencing that makes sequence based gene expression analysis a "digital" alternative to analog techniques. Target counting from next-generation DNA sequencing data is becoming increasingly attractive as the cost of DNA sequencing keeps decreasing, and is currently used for instance in NIPT screening. However, the current approach suffers from high sequencing library preparation costs and sequencing efforts that is wasted on sequencing non-relevant genetic targets. For instance, in cancer-related liquid biopsies, non-targeted approaches result in wastage of sequencing effort on oncologically non-relevant loci. In fetal diagnostics, non-targeted sampling of loci considerably limits the statistical options for interpreting the data. Guardant Health Inc provides more targeted sequencing approach, where an array of RNA capture probes enriches targets for next-generation DNA sequencing. Akhras et al. (2007) PLoS ONE 2(2):e223 disclose a multiplex pathogen detection assay involving barcoded target-specific probes, target circularization and sequencing. Use of a bridging oligonucleotide to ligate the target-specific probes is also disclosed.

Therefore, in light of the foregoing discussion, there exists a need to overcome the aforementioned drawbacks such as, but not limited to, specificity, sensitivity, accuracy, throughput, cost, scaling and turn-over through an accurate and massively parallel quantification of nucleic acid targets.

### SUMMARY OF THE INVENTION

The present disclosure seeks to provide a method for determining the Multiplexed Ligation Assay (MLA). The Multiplexed Ligation Assay is a molecular ligation-based assay that aims to combine the simplicity and parallel potential of the barcoding-MLPA with the accuracy and sensitivity of qPCR.

The disclosure relates more particularly to a method for the high-throughput detection of target nucleotide sequence detection in a very large number of samples by leveraging ligation-dependent assays. In an embodiment the target nucleotide sequence may be DNA or RNA, preferably DNA. In an embodiment, the methods include the steps of analyzing the target using high-throughput sequencing in the determination of the presence or absence and/or quantification of at least one target sequences in a plurality of samples.

In one aspect, the embodiment of the present disclosure provides the method, which further includes a set of target specific probes for each target nucleotide sequence in each of the samples. The designing of target specific probe assembly includes two target-specific nucleic acid probes per genetic target (left probe and right probe) or alternatively designing of target specific probe assembly with three target-specific nucleic acid probes of which two are specific for a genetic target (left probe and right probe) and one universal (bridge oligo). Universal (bridge oligo) is used for joining the left probe and the right probe.

In an embodiment the first probe includes, starting from the 5' end of the molecule, optionally a 5' phosphate, a first bridge oligo-specific sequence, optionally a first universal sequence, optionally a first sequence barcode, and a first target specific portion at 3' end of first probe. The second probe includes, starting from the 5' end of the molecule, optionally a 5' phosphate, a second target specific portion, optionally a second sequence barcode, optionally second universal sequence, and a second bridge oligo-specific sequence at 3' end of second probe. Preferably, at least one of the first sequence barcode or the second sequence barcode is present in the first probe or the second probe, respectively, wherein the first sequence barcode or the second sequence barcodes, or both, may be random sequences or may contain a target nucleotide sequence identifier sequences, a sample identifier sequences and/or molecular barcodes for target enumeration.

Preferably, the universal bridge oligo probe contains a promoter sequence for T7 RNA polymerase, sequences complementary to the first bridge oligo specific sequences and second bridge oligo-specific sequences in the first probe and the second probe, respectively, and optionally a third barcode which may be a random sequence or may contain a sample identifier sequence or a target identifier sequence. It is preferred that the promoter sequence of the T7 RNA polymerase is present in the bridge oligo, but instead of the promoter being present in the bridge oligo, it can also be present in the first or second probe. However, in such case, the design of the probes and oligo must be such that a T7 RNA polymerase is capable of transcribing all sequences that are necessary for identification of the sample and the target, and enumeration of the target sequences.

The first probe and the second probe include the first target specific portion, the second target specific portion, the first bridge oligo-specific sequences, and/or the second bridge oligo-specific sequences. The first probe and second probe contain sequences independently from one another. The first probe and the second probes are preferably chemically modified with one or more chemically modified nucleotide sequences.

The first probe and the second probe are contacted, preferably for each of the samples in a separate tube, with the bridge oligo. The plurality of the first and second probes self-anneal to the plurality of bridge oligos into a plurality of ligation complexes. Preferably each ligation complex is unique for the combination of the first target specific sequence, the second target specific sequence and one or more barcode sequences. This enables enumeration of the target sequences after amplification and analysis of the results.

Subsequently, the one or more target nucleotide sequence(s) in each sample is brought into contact with the ligation complexes allowing to self-anneal into a plurality of hybridized ligation complexes. The first target specific portion and the second target specific portion of the respective first probe and the second probe hybridize to essentially adjacent sections on the target sequence, thereby forming a hybridization complex. The ligation of the first probe and the second probe in the formed hybridized complexes is carried out either enzymatically or chemically to provide ligated ligation complexes. The ligated ligation complexes in the one or more samples are then subsequently pooled.

Optionally, and if present, a gap between the first probe and the second probe is filled by introducing a polymerase and one or more nucleotides. The polymerase adds nucleotides (a) complimentary to the universal bridge oligo sequence and (b) complimentary to the target sequence and thereby fills in the two gaps between the first probe and the second probe resulting in ligated the left and the right probe. The bridge oligo is extended from the 5' site or the 3' site complimentary to the ligated probes such that the target sequence identifier sequence is integrated into the bridge oligo, thereby forming one or more ligated ligation complex

In order to prevent the polymerase from extending the bridge oligo into the respective target sequences on the first and second probe before the first and second probe are ligated at the target sequence portion, preferably a suitable polymerase is used, more preferably a polymerase that does not extend into a double stranded DNA sequence. A typical example of such a polymerase is a Taq polymerase.

Next, T7 RNA polymerase that binds on the double stranded T7 RNA polymerase promoter site on the bridge oligo sequence of the ligated ligation complex and transcribes RNA downstream. Amplification of the synthesized RNA from the one or more ligated ligation complexes is carried out, for instance by using emulsion reverse transcriptase (RT)-PCR or T7 RNA polymerase. The free sample and probe nucleic acid removal is carried out by optionally adding a DNA-specific exonucleases and endonucleases to the pooled amplification reaction after RNA synthesis by T7 RNA polymerase but before cDNA synthesis or RT-PCR.

Preferably, the purification process of the ligated fragments is carried out by removing constituents of the ligation reaction mix using purification column or bead purification. Optionally, the cDNA is prepared from the RNA molecules using a DNA-oligonucleotide molecule that is reverse-complementary to a universal site 2. After cDNA is prepared, a conventional or, preferably, an emulsion PCR may be used to increase the signal. Alternatively RT-PCR or emulsion RT-PCR may be used.

The presence/ absence of the target nucleotide sequence is determined by subjecting the amplified RNA or cDNA molecules to high-throughput sequencing technology to determine the identifier sequence(s).

In another aspect, an embodiment of the present disclosure provides a kit for use in the methods of the invention. The kit includes a plurality of containers, wherein at least one container includes one set of a first probe and a second probe, and wherein at least one container comprises a bridge oligo. In one particular embodiment, the at least one container comprising the set of first and second probe and the at least one container comprising the bridge oligo are one and the same container. In such case, the three probes may be pre-annealed and have formed a ligated complex.

One particular advantage of the invention is to enable the detection and amplification of target sequence of interest using unique probe designs i.e. probe triplet. The probes are designed with improved binding properties and lead to higher assay specificity, sensitivity and accuracy. The present invention finds application in the area of molecular biology, evolutionary biology, metagenomics, genotyping and more specifically but not limited to cancer diagnostics, and fetal chromosomal disorders.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. Those in the art will understand that the drawings are not to scale. Wherever possible, like elements have been indicated by identical numbers.

Embodiments of the present disclosure will now be described, by way of example only, with reference to the following diagrams wherein:
FIG. 1 illustrates a flow diagram of the Multiplexed Ligation Assay (MLA) according to an embodiment herein;
FIG. 2A illustrates a principle structure of probes triplet having a plurality of probe entities according to an embodiment herein;
FIG. 2B illustrates the gap filling between the first probe and the second probe according to an embodiment herein;
FIG. 3 shows possible chemical modifications that increase probe binding efficiency and improve ligation efficiency;
FIG. 4 provides an overview of analysis of replicated chromosomal count data according to an embodiment herein;
FIG. 5 provides an overview for quantitatively identifying the proportion of mutated variants of multiple genes according to an embodiment herein; and
FIG. 6 illustrates a flowchart of multiplex ligation dependent probe amplification according to an embodiment herein.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

In a first aspect, the invention provides a method for the high-throughput detection of one or more target nucleotide sequence in a plurality of samples, the method comprising the steps of:
(i) providing for each target nucleotide sequence in each of the samples:
   a first probe, a second probe and a bridge oligo,
   wherein the first probe comprises, starting from the 5' end of the molecule, a first bridge oligo-specific sequence, optionally a first sequence barcode, and a first target specific portion at the 3'end of first probe;
   and wherein the second probe comprises, starting from the 5' end of the molecule, a second target specific portion, optionally a second sequence barcode, and a second bridge oligo-specific sequence at the 3' end of second probe;
   and wherein the bridge oligo contains sequences complementary to the first bridge oligo specific sequence and the second bridge oligo-specific sequence in the first probe and the second probe, respectively, and optionally a third barcode;
   wherein at least one of the first sequence barcode or the second sequence barcode or the third barcode is present in the first probe or the second probe or the bridge oligo, respectively; and
   wherein a promotor sequence for a T7 RNA polymerase is present in the first probe, the second probe or the bridge oligo,
(ii) contacting, for each of the one or more target nucleotide sequence, the first probe and the second probe with, preferably for each of the samples in a separate tube, the bridge oligo and allow self-annealing into a plurality of ligation complexes;
(iii) contacting nucleic acids present in the plurality of samples to be tested for the target nucleotide sequences with the plurality of ligation complexes;
(iv) allowing the first target specific portion and the second target specific portion of the respective first probe and the second probe to hybridize to essentially adjacent sections on the target sequence, thereby forming a hybridization complex;
(v) ligating the first probe and the second probe in the hybridization complexes to provide ligated ligation complexes,
(vi) pooling the ligated ligation complexes from the plurality of samples,
(viii) amplifying RNA from the one or more ligated complexes using T7 RNA polymerase that initiates RNA synthesis from the T7 RNA polymerase promoter embedded in the ligated ligation complexes;
(xi) optionally, preparing cDNA from the RNA molecules using a DNA-oligonucleotide molecule that is reverse-complementary to a universal site 2;
(xii) subjecting the RNA or the cDNA molecules to high-throughput sequencing technology to determine the identifier sequence(s); and
(xiii) identifying the presence and/or number of the target nucleotide sequence in the plurality of samples by determination of at least part of the first target specific portion and/or the second target specific portion, and/or at least part of the first barcode and/or the second barcode, and/or at least part of the third barcode.

In a first embodiment, the method comprises the steps of:
(i) providing for each target nucleotide sequence in each of the samples: a first probe, a second probe and a bridge oligo, wherein
   the first probe comprises, starting from the 5' end of the molecule, optionally a 5' phosphate, a first bridge oligo-specific sequence, optionally a first universal sequence, optionally a first sequence barcode, and a first target specific portion at the 3' end of the first probe; and wherein
   the second probe comprises, starting from 5' end of the molecule, optionally a 5' phosphate, a second target specific portion, optionally a second sequence barcode, optionally a second universal sequence, and a second bridge oligo-specific sequence at the 3' end of the second probe; and wherein the bridge oligo includes a sequences complementary to the first bridge oligo-specific sequence and the second bridge oligo-specific sequence in the first probe and the second probe, respectively, and optionally a third barcode, which may be a random sequence or may contain a sample identifier sequence or a target identifier sequence; and wherein
   at least one of the first sequence barcode or the second sequence barcode or the third barcode is present in the first probe or the second probe or the bridge oligo, respectively, wherein the first sequence barcode or the second sequence barcode or both, may be random sequences or may contain a target nucleotide sequence identifier sequences, a sample identifier sequences and/or molecular barcodes for target enumeration; and wherein
   a promotor sequence for a T7 RNA polymerase is present in the first probe, the second probe or the bridge oligo, and wherein preferably the first target specific portion, the second target specific portion, the first bridge oligo-specific sequences, and/or the second bridge oligo-specific sequences include, independently from one another, one or more chemically modified nucleotides;
(ii) contacting for each of the one or more target nucleotide sequences, the first probe and the second probe with, preferably for each of the samples in a separate tube, the bridge oligo and allow self-annealing into a plurality of ligation complexes;
(iii) contacting nucleic acids present in the plurality of samples to be tested for the target nucleotide sequences with the plurality of ligation complexes;
(iv) allowing the first target specific portion and the second target specific portion of the respective first probe and the second probe to hybridize to essentially adjacent sections on the target sequence, thereby forming a hybridization complex;
(v) ligating the first probe and the second probe in the hybridization complexes to provide ligated ligation complexes;
(vi) pooling the ligated ligation complexes from the plurality of samples;
(vii) optionally, providing to the hybridization complexes a polymerase and one or more nucleotides, such that a gap present between the first probe and the second probe on the bridge oligo is filled-in, such that the ligated probes are extended from the 5' site or the 3' site such that a barcode sequence present in the bridge oligo integrates into the ligated probe, or such that the bridge oligo is extended from the 5' site or the 3' site such that a barcode sequence present in the first probe or the second probe integrates into the bridge oligo, or both, thereby forming one or more ligated ligation complexes;
(viii) amplifying RNA from the one or more ligated ligation complexes, using the T7 RNA polymerase that initiates RNA synthesis from the T7 RNA polymerase promoter embedded in the ligated complexes;
(ix) optionally, adding DNA-specific exonucleases and endonucleases to the pooled amplification reaction to remove sample and probe DNA;
(x) optionally, purifying the remaining RNA using bead or column-cleanup;
(xi) optionally, preparing cDNA from the RNA molecules using a DNA-oligonucleotide molecule that is reverse-complementary to the second universal site;
(xii) subjecting the amplified RNA or cDNA molecules to high-throughput sequencing technology to determine the identifier sequence(s); and
(xiii) identifying the presence and/or number of the target nucleotide sequence in the plurality of samples by determination of at least part of the first target specific portion and/or the second target specific portion, at least part of the first barcode sequence and/or the second barcode sequence, and/or at least part of the third barcode sequence.

### Definitions:

Target Nucleotide Sequence: The term target nucleotide sequence may be any nucleotide sequence of interest of which its detection is required. It will be understood that the term given refers to a sequence of contiguous nucleotides as well as to nucleic acid molecules with the complementary sequence. The target sequence preferably is a nucleotide sequence that represents or is associated with a polymorphism.

Polymorphism: The term polymorphism refers to an occurrence of two or more genetically determined alternative sequences or alleles in a population. A polymorphic marker or site is the locus at which sequence divergence occurs. A polymorphic locus may be as small as one base pair.

Samples: The term samples is used herein for two or more samples which contain two or more target sequences. Samples as provided in a method according to the invention have preferably been prepared in order to extract at least the target nucleic acids and make those accessible to the probes as used in the invention. In particular, the samples each comprises at least two different target sequences, preferably at least 100, more preferably at least 250, more preferably at least 500, most preferably at least 2000, or more. The term samples may refer to but is not limited to two or more samples obtained from a human body, including biopsies, saliva and other secretions, exhaled moisture extracts, tissue, blood plasma (liquid biopsies), two or more samples obtained from environment, including water, wastewater, soil, or the like.

Probe: The term probe is a fragment of DNA or RNA of variable length (usually 50-1000 bases long, preferably 50 - 200 bases long) which can be used in DNA or RNA samples to detect the presence of nucleotide sequences (the DNA or RNA target) that are complementary to the sequence in the probe. The sections of the oligonucleotide probes that are complementary to the target sequence are designed such that for each target sequence in a sample, a pair of a left and a right probe is provided, whereby the probes each contain a section at their extreme end that is complementary to a part of the target sequence. Furthermore, the present disclosure describes a bridge oligo that is used for joining the left probe and the right probe.

Universal: The term universal when used to describe an amplification procedure refers to a sequence that enables the use of a single primer or set of primers for a plurality of amplification reactions. The use of such primers greatly simplifies multiplexing in that only two primers are needed to amplify a plurality of selected nucleic acid sequences. The term universal when used to describe a priming site is a site to which a universal primer will hybridize. It should also be noted that "sets" of universal priming sequences/primers may be used.

Hybridization: The term hybridization (or hybridisation) describes the process of deoxyribonucleic acid (DNA) or ribonucleic acid (RNA) molecules annealing to complementary DNA or RNA. DNA or RNA replication and transcription of DNA into RNA both rely on nucleotide hybridization.

Ligation: The term ligation is the joining of two nucleic acid fragments through the action of an enzyme. DNA ligases are enzymes capable of catalyzing the formation of a phosphodiester bond between (the ends of) two polynucleotide strands bound at adjacent sites on a complementary strand. Ligation can also be performed chemically, in particular if both adjacent ends of the polynucleotides are modified to allow chemical ligation.

Amplification: The term amplification as used herein denotes the use of polymerase chain reaction (PCR) to increase the concentration of a particular nucleotide sequence within a mixture of nucleotide sequences. "PCR" or "Polymerase Chain Reaction" is a rapid procedure for in vitro enzymatic amplification of a specific DNA/RNA segment. The DNA/RNA to be amplified is denatured by heating the sample. The term primer is a short strand of RNA or DNA (generally about 18-22 bases) that serves as a starting point for DNA synthesis. It is required for DNA replication because the enzymes that catalyze this process, DNA polymerases, can only add new nucleotides to an existing strand of DNA. A T7 RNA polymerase is capable of both, transcribing and amplifying a single DNA molecule into multiple copies of RNA, which may be converted back to cDNA.

Polymerase: A polymerase is an enzyme that synthesizes long chains or polymers of nucleic acids. DNA polymerase and RNA polymerase are used to assemble DNA and RNA molecules, respectively, by copying a DNA or RNA template strand using base-pairing interactions. A specific polymerase used herein, T7 RNA Polymerase, is a RNA polymerase from the T7 bacteriophage that catalyzes the formation of RNA in the 5'→ 3' direction. The T7 RNA polymerase requires a partially double stranded DNA template and Mg2+ ion as cofactor for the synthesis of RNA. A T7 RNA polymerase is capable of both, transcribing and amplifying a single DNA molecule into multiple copies of RNA.

High throughput: The term high throughput denotes the ability to simultaneously process and screen a large number of DNA samples; as well as to simultaneously screen large numbers of different genetic loci within a single DNA sample. High-throughput sequencing or screening, often abbreviated as HTS is a method for scientific experimentation especially relevant to effectively screen large amounts of samples simultaneously.

Uracil Specific Excision Reagent (USER): Uracil Specific Excision Reagent also known as USER permits linearization of a circular DNA molecule by cleavage where a deoxyuridine nucleotide is present.

The disclosure provides a method for determining the sequences of genetic targets in complex nucleic acid pools using techniques permitted by next generation sequencing. The disclosure provides a method to profile multiple genetic targets in a number of samples, preferably a very large number of samples, by leveraging ligation-dependent assays. The disclosure provides a method for the multiplex ligation-dependent probe amplification enabling querying different target nucleic acids in a plurality of samples. The present disclosure provides a method for the sequencing of target nucleic acid for those skilled in art with accurate and massively parallel quantification of nucleic acid targets of interest that are present in a sample, for the detection of the presence and absence of genetic variants. Furthermore, unique sequence identifiers are used for the identification of the genetic targets and absolute quantification of individual samples from the sample pool when processing the sequencing data.

In a preferred embodiment, the method of the present invention is related to the identification of the plurality of samples of nucleic acid using high throughput sequencing technologies. The methods of the present invention allow the sequencing of the one or more target nucleotide sequence in a plurality of samples providing a plurality of different probe sets for different target nucleic acids. The methods of the present invention utilize three nucleic acid probes, out of which two target-specific nucleic acid probes (left probe and right probe) are specific for a genetic target and one nucleic acid probe is universal (bridge oligo). The left and right probe hybridize to the bridge probe, forming a ligation complex. The ligation complex (containing one or more barcode sequences) having target identification sites on the sample DNA/ RNA are allowed to hybridize against complementary target sequence of the query sample under ambient conditions. After hybridization the left and right probe ligate chemically or enzymatically by a DNA ligase to form ligated ligation complex. In present invention, a plurality of such ligated ligation complexes will form during the sample analysis in the plurality of samples to be analyzed.

A T7 RNA polymerase amplifies the joined probes to be used for sequencing using a next-generation sequencing platform including without limiting, Illumina MiSeq, HiSeq or NextSeq. The samples are separated (de-convoluted) from the sequence data and the sequence targets quantified *in silico* after the DNA sequencing. In a preferred embodiment, the sequencing is carried out by means of next-generation DNA or RNA sequencing.

In an embodiment, "plurality of samples" may refer to, but not limited to, two or more samples obtained from a human body, including biopsies, saliva and other secretions, exhaled moisture extracts, tissue, blood plasma (liquid biopsies), two or more samples obtained from environment, including water, wastewater, soil, or the like. The sample used may be DNA or RNA extracted using any of the currently available extraction technologies such as ethanol precipitation, phenol-chloroform extraction, silica column, or the like.

In an embodiment the methods include the steps of analyzing the target using high-throughput sequencing in the detection and/or quantification of at least one target sequences in the plurality of samples. The target sequence includes any nucleotide sequence of interest against which the detection is required. The target nucleotide sequence of the disclosure is obtained from, but not limited to, a fraction of DNA in the patient's blood or a fraction of DNA in maternal blood. A fraction of the DNA in the patient's blood is obtained from apoptotic/necrotic cancer cells or a fraction of DNA in maternal blood from fetal origin. Further, the results of analysis are used to, for instance, assess the risk of an individual to a given type of cancer, the determine the efficacy of a given treatment against a given cancer, the development of a drug-resistance-related mutations in a tumor, or the risk of a fetus carrying genetic disorders such as common trisomies Down, Patau and Edwards syndromes.

In an embodiment a method for the high throughput detection of one or more target nucleotide sequences is provided that may be derived from one or more samples. In certain embodiments, the method comprises providing, for each target nucleotide sequence, a plurality of different probe sets. As used herein, the term probe sets includes a first probe, a second probe and a bridge oligo.

In certain embodiments, the first probe includes, starting from the 5' end of the molecule, optionally a 5' phosphate, a first bridge oligo-specific sequence, optionally a first universal sequence, optionally a first sequence barcode, and a first target specific portion at its 3'end. In certain embodiments, the second probe includes, starting from 5' end of the molecule, optionally a 5' phosphate a second target specific portion, optionally a second sequence barcode, optionally a second universal sequence, and second bridge oligo-specific sequence at its 3'end.

In a preferred embodiment, either the first probe or the second probe contain at least one of the first sequence barcode or the second sequence barcode. The first sequence barcode or the second sequence barcode, or both, may be random sequences or may contain target nucleotide sequence identifier sequences, sample identifier sequences and/or molecular barcodes for target enumeration.

In preferred embodiments the bridge oligo contains a promoter sequence for T7 RNA polymerase, sequences complementary to the first and second bridge oligo-specific sequences in the first and second probe, respectively, a universal sequence, and/or or may contain a third barcode which may be a random sequence or may contain a sample or sequence identifier sequence. In this respect, third barcode does not necessarily mean that there are already a first and a second barcode present. As described earlier, at least one barcode should be present in the ligated ligation complex, that enables to uniquely define the complex within all ligation complexes in all samples tested.

The first target specific portion, the second target specific portion, the first bridge oligo-specific sequences, and/or the second bridge oligo-specific sequences, preferably contain independently from one another at least one chemically modified nucleotide. The chemical modifications that increase probe binding include, but are not limited to, ribonucleic acids, peptide nucleic acids, and locked nucleic acids. In certain embodiments, chemical modifications permit chemical ligation of adjacent probes. The aforementioned probes bind to completely adjacent genetic loci or up to 50 base pairs apart, preferably up to 40 base pairs apart, more preferably up to 30 base pairs apart, more preferably up to 20 base pairs apart, more preferably up to 10 base pairs apart, most preferably up to 5 base pairs apart.

Before contacting the probes with the sample comprising the target sequences, the first probe and the second probe is brought in contact with the bridge oligo, preferably for each of the samples in a separate tube, and self-annealing into ligation complexes is allowed. Thereafter, the one or more target nucleotide sequences in the plurality of samples is brought into contact with the plurality of ligation complexes. The first target specific portion and the second target specific portion of the respective first probe and the second probe hybridize to essentially adjacent sections on the target sequence, thereby forming a hybridization complex. The ligation of the first probe and the second probe in the formed hybridized complexes is carried out either enzymatically or chemically to provide ligated ligation complexes. The ligated ligation complexes are then subsequently pooled from one or more target samples.

The gap between the first probe and the second probe, if present, may be filled by introducing a polymerase and one or more nucleotides. The polymerase adds nucleotides (a) complimentary to the universal bridge oligo sequence and/or (b) complimentary to the barcode sequence and thereby fills in the two gaps between the first probe and the second probe resulting in ligated the left and the right probe and inclusion of the universal sequence and/or third barcode sequence into the bridge complementary strand. The bridge oligo is extended from the 5' site or the 3' site complimentary to the ligated probes such that the target sequence identifier sequence present in the first probe or second probe is integrated into the bridge oligo. Preferably, a polymerase is used that does not break up double stranded DNA, such as for instance a Taq polymerase, in order not to interfere with the ligation of the first to the second probe when both are annealed to the target sequence.

Preferably, either of the probes or the bridge oligo contains a deoxyuridine moiety that permits linearization by cleavage using uracil-specific excision reagent. This enables transcribing RNA from the linearized ligated ligation complexes using T7 RNA polymerase that initiates RNA synthesis from the T7 RNA polymerase promoter embedded in the bridge oligo or the first or second probe. After amplification, the free sample and probe nucleic acid removal is carried out by optionally adding a DNA-specific exo-nucleases and endonucleases to the pooled amplification reaction. As said before, the T7 RNA polymerase promotor sequence may be situated in the bridge oligo, the first probe or in the second probe. The same holds true for the deoxyuridine moiety. However, as is clear for the skilled person, the T7 RNA polymerase promotor sequence and the deoxyuridine moiety should be situated such that the T7 RNA polymerase is capable of transcribing all information that is necessary for the enumeration of the different targets in the different samples. Between T7 RNA polymerase and deoxyuridine moiety the following sequences should occur: at least one sequence that enables identifying the target, at least one sequence that enables identifying the sample and at least one unique identifier sequence that enables determining the number of copies of the target sequence in the sample.

A purification process of the ligated fragments may be carried out by removing constituents of the ligation reaction mix using purification column or bead purification. Optionally, the cDNA is prepared from the RNA molecules using a DNA-oligonucleotide molecule that is reverse-complementary to a universal site 2. The RNA molecules are optionally converted to cDNA and optionally amplified by PCR or emulsion PCR by using primers binding to the universal parts of the probes.

The identification of the presence and/or number of the target nucleotide sequence in the plurality of samples by determination of at least part of the first and/or second target specific portion, at least part of the first and/or second barcode, and/or at least part of the third barcode by high-throughput sequencing technology. Preferably, the genetic target enumeration is permitted by counting the number of molecular barcodes per target and per sample.

The presence/ absence of the target nucleotide sequence is determined by subjecting the amplified RNA or cDNA molecules to high-throughput sequencing technology to determine the identifier sequence(s). In a preferred embodiment, the RNA molecules or the cDNA molecules are amplified with a first primer and a second primer to provide an amplification product. Preferably a universal first primer and a universal second primer are used, complementary to a first and second universal sequence present in the ligated complexes.

The methods of the invention involve various aspects that are advantageous when compared to the methods used in prior art for nucleotide sequencing and amplification. The methods as disclosed in the invention aim to provide an assay that is better than any of the existing target, without limiting Quantitative PCR (qPCR), Array PCR, Digital PCR, Mutiplex-Ligation-Dependent Probe Amplification or the like.

The advantages of the present invention include, but are not limited to quantification assay with low cost, high simplicity, high specificity, high sensitivity, high accuracy, high throughput, high scalability and high turn-over in comparison to traditional nucleic acid sequencing technologies. Another aspect of the present invention is that the methods of the present invention allow accurate and massively parallel quantification of plurality of nucleic acid targets in multiple samples including human and animal populations. One particular advantage of the invention is to enable the detection and amplification of target sequence of interest using unique probe designs, i.e., probe triplet. The probes are designed with specially situated modified nucleotides that improve annealing and binding efficiency. Improvement in binding properties leads to higher assay specificity, sensitivity and accuracy. The methods of the present invention are likewise applicable for studying genetic variants and find application in diagnosis and prognosis, including but not limited to genotype the sample(s) for one or more sequences and/or polymorphisms, such as SNPs and/or indels, cancer diagnostics or fetal chromosomal disorders from maternal blood. In a preferred embodiment, for two or more samples or for two or more locus/allele combinations, barcode sequences are used to genotype the samples for one or more sequences and/or polymorphisms, such as SNPs and/or indels.

In another aspect, the invention provides a kit of parts comprising a plurality of containers, wherein at least one container comprises one or more sets of first probe and second probe, and at least one container comprises one or more bridge oligos,
wherein the first probe comprises, starting from the 5' end of the molecule, a first bridge oligo-specific sequence, optionally a first sequence barcode, and a first target specific portion at 3' end of first probe;
wherein the second probe comprises, starting from the 5' end of the molecule, a second target specific portion, optionally a second sequence barcode, and a second bridge oligo-specific sequence at 3' end of second probe;
wherein the bridge oligo comprises sequences complementary to the first and second bridge oligo-specific sequences in the first and second probe, respectively, and optionally a third barcode;
and wherein at least one of the first sequence barcode or the second sequence barcode or the third barcode is present in the first probe or the second probe or the bridge oligo, respectively;
and wherein a promotor sequence for a T7 RNA polymerase is present in the first probe, the second probe or the bridge oligo,

In one embodiment, the kit of parts includes a plurality of container, wherein at least one container comprises one or more sets of first probe second probe, and at least one container comprises one or more bridge oligos,
wherein the first probe comprises, starting from the 5' end of the molecule, optionally a 5' phosphate, a first bridge oligo-specific sequence, optionally a first universal sequence, optionally a first sequence barcode, and a first target specific portion at the 3' end of the first probe;
wherein the second probe comprises, starting from the 5' end of the molecule, optionally a 5' phosphate, a second target specific portion, optionally a second sequence barcode, optionally a second universal sequence, and a second bridge oligo-specific sequence at the 3'end of the second probe;
wherein the bridge oligo comprises sequences complementary to the first bridge oligo-specific sequence and second bridge oligo-specific sequence in the first probe and the second probe, respectively, and optionally a third barcode which may be a random sequence or may contain a target nucleotide identifier sequence or a sample identifier sequences; and
wherein at least one of the first sequence barcode or the second sequence barcode or the third barcode is present in the first probe or the second probe or the bridge oligo, respectively, wherein the first sequence barcode or the second sequence barcode or both, may be random sequences or may contain a target nucleotide sequence identifier sequences, a sample identifier sequences and/or molecular barcodes for target enumeration; and wherein a promotor sequence for a T7 RNA polymerase is present in the first probe, the second probe or the bridge oligo,
wherein preferably the first target specific portion, the second target specific portion, the first bridge oligo-specific sequence, and/or the second oligo-specific sequence may include, independently from one another, one or more chemically modified nucleotides.

Preferably, the 3' end of the first probes or the 5' end of the second probes, or both, are modified to permit chemical ligation of the first probes to the second probes.

Preferably, the bridge oligo comprises one or more chemically modified nucleotides in the sequence complementary to a sequence of the first probe or in the sequence complementary to a sequence of the second probe, or both.

Preferably, the first probe, the second probe, or the bridge oligo comprises a deoxyuridine moiety that permits linearization by cleavage using uracil-specific excision reagent.

Preferably, the 3' end of the first probe or the 5' end of the second probe, or both, are modified to permit chemical ligation of the first probe to the second probe.

Preferably, the bridging portion of the first probe or the second probe, or both, comprise(s) chemically modified bases to permit improved binding to the bridge oligo.

In another aspect, the invention provides a use of a first probe, a second probe and a bridge oligo for high-throughput sequencing in the determination of the presence or absence and/or quantification of one or more target sequences in a plurality of samples,
wherein the first probe comprises, starting from the 5' end of the molecule, a first bridge oligo-specific sequence, optionally a first sequence barcode, and a first target specific portion at 3' end of first probe;
and wherein the second probe comprises, starting from the 5' end of the molecule, a second target specific portion, optionally a second sequence barcode, and a second bridge oligo-specific sequence at 3'end of the second probe;
and wherein the bridge oligo comprises sequences complementary to the first bridge oligo-specific sequences and the second bridge oligo-specific sequences in the first probe and the second probe, respectively, and optionally a third barcode;
wherein at least one of the first sequence barcode or the second sequence barcode or the third barcode is present in the first probe or the second probe or the bridge oligo, respectively; and wherein a promotor sequence for a T7 RNA polymerase is present in the first probe, the second probe or the bridge oligo; and wherein the first probe and the second probe are ligated when the respective target specific sections of the probes are hybridized to essentially adjacent sections on the target sequence to provide ligated probes; and the ligated probes from the plurality of samples are pooled and amplified using T7 RNA polymerase; and the amplification products are subjected to high-throughput sequencing technology to determine the identifier sequence(s).

The first probe comprises, starting from the 5' end of the molecule, optionally a 5' phosphate, a first bridge oligo-specific sequence, optionally a first universal sequence, optionally a first sequence barcode, and a first target specific portion at the 3' end of the first probe.

The second probe comprises, starting from the 5' end of the molecule, optionally a 5' phosphate, a second target specific portion, optionally a second sequence barcode, optionally a second universal sequence, and a second bridge oligo-specific sequence at the 3' end of the second probe.

The bridge oligo includes sequences complementary to the first bridge oligo-specific sequence and the second bridge oligo-specific sequence in the first probe and the second probe, respectively, and optionally a third barcode which may be a random sequence or may contain a sample identifier sequence.

At least one of the first sequence barcode or the second sequence barcode or the third barcode is present in the first probe or the second probe or the bridge oligo, respectively, wherein the first sequence barcode or the second sequence barcode or both, may be random sequences or may contain a target nucleotide sequence identifier sequences, a sample identifier sequences and/or molecular barcodes for target enumeration; and wherein a promotor sequence for a T7 RNA polymerase is present in the first probe, the second probe or the bridge oligo.

The first target specific portion, the second target specific portion, the first bridge oligo-specific sequence, and/or the second bridge oligo-specific sequence preferably contain, independently from one another, one or more chemically modified nucleotide.

The first probe and the second probe ligate when the respective target specific sections of the probes hybridize to essentially adjacent sections on the target sequence to provide ligated probes. The T7 RNA polymerase is used for the amplification of ligated probes from the plurality of samples, preferably after pooling the plurality of samples. The amplification products are further subjected to high-throughput sequencing technology to determine the identifier sequence(s).

Preferably, the 3' end of the first probes or the 5' end of the second probes, or both, are modified to permit chemical ligation of the first probes to the second probes.

Preferably, the bridge oligo comprises one or more chemically modified nucleotides in the sequence complementary to a sequence of the first probe or in the sequence complementary to a sequence of the second probe, or both.

Preferably, the first probe, the second probe, or the bridge oligo comprises a deoxyuridine moiety that permits linearization by cleavage using uracil-specific excision reagent.

Preferably, the 3' end of the first probe or the 5' end of the second probe, or both, are modified to permit chemical ligation of the first probe to the second probe.

Preferably, the bridging portion of the first probe or the second probe, or both, comprise(s) chemically modified bases to permit improved binding to the bridge oligo.

One particular advantage of the invention is to enable the detection and amplification of target sequence of interest using unique probe designs i.e. probe triplet. The probes are designed with improved binding properties lead to higher assay specificity, sensitivity and accuracy. The present invention finds application in cancer diagnostics or fetal chromosomal disorders but not limited to genotype the sample(s) for one or more sequences and/or polymorphisms, such as SNPs and/or indels.

In one particular preferred embodiment, the bridge oligo comprises information to identify the sample and includes a unique identifier. In such case, the first and second probe is universally applicable to all samples (and only comprises information to identify the target). In one preferred embodiment, therefore, a method, a kit or a use according to the invention is provided, wherein the bridge oligo comprises a barcode that comprises a unique sequence that enables enumeration of the target sequences of each sample.

### EXPERIMENTAL SECTION

The following experiments provide exemplifications of the various embodiments of the invention.

Case 1: An aged person suffering from an advanced lung cancer is pretreated with chemotherapy as the first line of treatment. Analysis of the tumor by tissue biopsy is not applicable due to chemotherapy and the condition of the patient. Thus, there are no possibilities for obtaining the tissue DNA from the tumor of the patient. For second line treatment, a Guardant360 liquid biopsy is performed and, on basis of the results, the patient has a targeted treatment for cancer. For the follow-up, the targeted mutation and related resistance mutations should be followed. Cost of the treatment is €7000/month, and weekly samples would be optimal for the detection of the possible resistance for the used drug. If an individualized targeted mutation analysis (with rapid TA-time) could be performed non-invasively right at the time of developed resistance, the new wide-range liquid biopsy could be considered, and a new targeted drug could be considered.

The savings of omitting the resistance developed drug with no longer effect is €7-€21000 at 1-3 months of the treatment. Further, the benefits of the next generation treatment options are achieved faster and may improve the outcome of the disease.

Case 2: Multiplexed ligation assay to detect fetal chromosomal trisomies in maternal blood.

Each relevant gene is targeted by multiple probes that can detect typical mutations. The multiple probes use permits quantitatively identifying the proportion of mutated variants of multiple genes. Typically, 10 distinct ligation complexes target each chromosome for the detection of fetal chromosomal disorders from maternal blood. The use of 10 distinct probes results in 10 independent replicates for each chromosome. The set of multiple ligation complexes detect significant mutations. The replicated chromosomal count data is analyzed using statistical tools to detect significant deviations from the standard deviation pattern of the chromosomal targets. The deviations from the standard deviation pattern of the chromosomal targets permits identifying deletions (such as Williams syndrome) and trisomies (such as Patau, Edwards or Down syndrome).

Case 3: Multiplexed ligation assay to detect oncologically relevant mutations in circulating DNA of a patient suffering from cancer.

For the detection of oncologically relevant gene mutations in circulating DNA from a patient suffering from cancer, each relevant gene is targeted by multiple ligation complexes that can detect mutations typical of the cancer type. Multiplexed ligation assay permits quantitatively identifying the proportion of mutated variants of multiple genes.

Case 4: Multiplexed ligation assay to detect and quantify multiple antibiotic resistance genes in fecal or wastewater samples.

For the detection of antibiotic resistance genes in DNA extracted from faecal or wastewater material, each antibiotic resistance gene is targeted by multiple specific ligation complexes. The use of several distinct ligation complexes per target gene results in independent technical replicates for each gene. This replicated count data is analyzed using statistical tools to quantify the copy number of a multiplicity of antibiotic resistance genes in a multiplicity of samples.

### DETAILED DESCRIPTION OF THE DRAWINGS

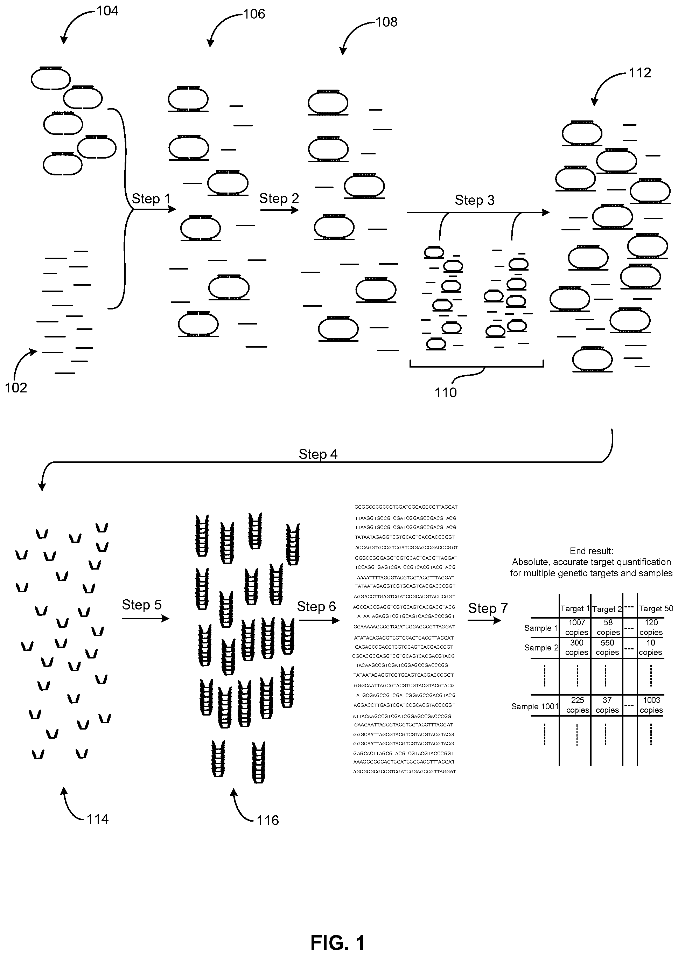

FIG 1 illustrates the workflow of one embodiment of the described invention. In step 1, nucleic acids (DNA or RNA) extracted from a sample (102) are brought into contact with a set of ligation complexes (104). The ligation complexes anneal on the target nucleic acids (106). In step 2, the annealed ligation complexes are ligated, resulting in ligated ligation complexes (108). In step 3, ligated ligation complexes from multiple samples (110) are pooled together (112). In step 4, RNA (114) is synthesized from the ligated ligation complexes using T7 RNA polymerase. Probe and sample DNA is optionally removed using a mixture of endo- and exonucleases. In step 5, the RNA is converted into cDNA (116) and optionally amplified using PCR or emulsion PCR. In step 6, the amplified DNA is sequenced using next-generation DNA sequencing. In step 7, the DNA sequencing results are converted into target counts using a bioinformatic pipeline.

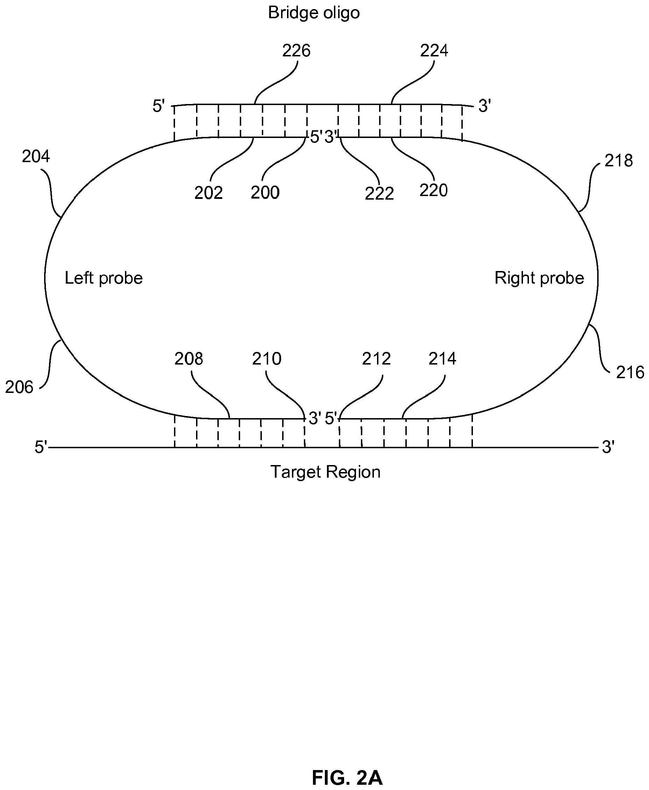

FIG. 2A illustrates a principle structure of a probe triplet having a plurality of probe entities according to an embodiment herein. The plurality of probe entities include a left probe, a right probe and a bridge oligo assembled prior to sample annealing. The first base of the left probe optionally includes a phosphate moiety for enzymatic ligation or a modification that permits chemical ligation to the 5' end of the adjacent probe referred to as modification 1 (200). 15-25 bases of the left probe includes an bridge binding sequence 1 (202), that further includes chemically modified bases for efficient bridge oligo binding referred as bridge site 1. Aforementioned segment also optionally includes reverse complementary sequence of the T7 RNA polymerase promoter. As said before, this promotor sequence may be present in the first probe or in the second probe, instead of in the bridge oligo, provided the oligo and probes are designed such that the T7 RNA polymerase is able to transcribe all necessary information for enumeration of the target sequences in the different samples. The following 15-30 bases of the left probe optionally include a universal binding site for PCR primers referred herein as universal site 1, (204). The left probe further optionally includes following 10-20 bases from the 5' end including segments of random nucleotides which form the molecule- specific barcode or sample-specific barcode referred to as barcode 1 (206). The left probe further includes following 15-30 bases from the 5' end, binds to the genetic target (208). Some or all of the nucleotides of 202 or 208 may include chemical modifications that increase the affinity of the probes to the target or the bridge oligo (226). The last base of the left probe optionally includes a phosphate moiety for enzymatic ligation or a modification that permits chemical ligation to the 5' end of the adjacent probe referred to as modification 1 (210).

The first base of the right probe optionally includes a phosphate moiety for enzymatic ligation or a modification that permits chemical ligation to the 5' end of the adjacent probe referred to as modification 2 (212). The 15-30 bases from the 5' end of the right probe include a part of the right probe that binds to the genetic target (214). The following 10-20 bases from the 5' end of the right probe optionally include segments of random nucleotides which form the molecule-specific barcode or sample-specific barcode referred to as barcode 2 (216). The following 15-30 bases from 5' end of the right probe optionally include a universal binding site for PCR primers referred to as universal site 2 (218). The last 15-25 bases of the right probe include an anchor sequence for efficient bridge oligo binding, referred as bridge sequence 2 (220). Aforementioned segment also optionally includes reverse complementary sequence of the T7 RNA polymerase promoter. As said before, this promotor sequence may be present in the first probe or in the second probe, instead of in the bridge oligo, provided the oligo and probes are designed such that the T7 RNA polymerase is able to transcribe all necessary information for enumeration of the target sequences in the different samples. Some or all of the nucleotides of 214 or 220 may include chemical modifications that increase the affinity of the probes to the target or the bridge oligo (224). The last base of the right probe optionally includes a phosphate moiety for enzymatic ligation or a modification that permits chemical ligation to the 5' end of the adjacent probe referred to as modification 2 (222).

The first 15-25 bases from the 5' end of the bridge oligo, referred as bridge sequence 3 (226), are reverse complementary to the bridge sequence 1 of the right probe (202), and optionally include chemically modified nucleotides for increased binding. The following 15-25 bases of the bridge, referred as bridge sequence 4 (224), are reverse complementary to the bridge sequence 2 sequence of the left probe (220), and optionally include chemically modified nucleotides for increased binding. Bridge sequence 3 (226) or bridge sequence 4 (224), optionally include the sequence of the T7 RNA polymerase promoter.

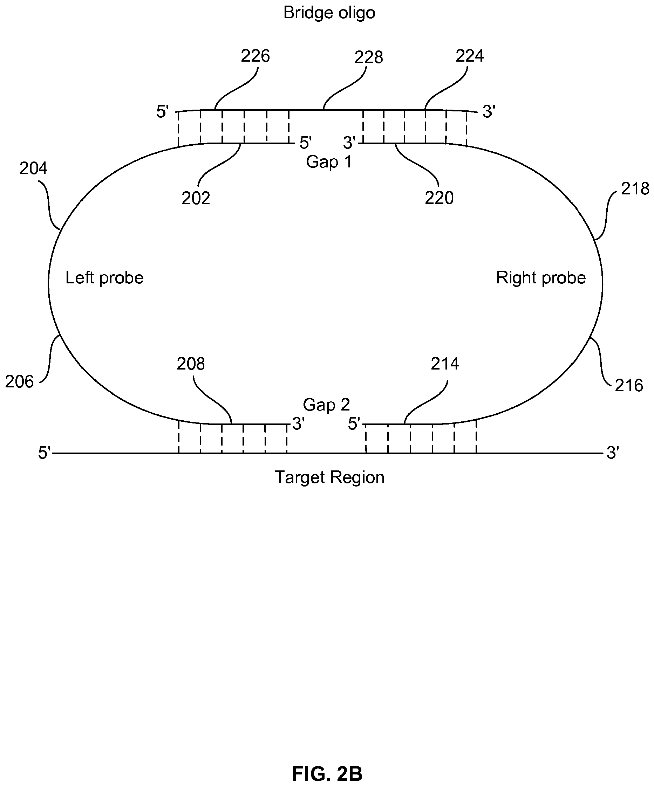

FIG. 2B illustrates gap filling between the first probe and the second probe according to an embodiment herein. Here, the bridge oligo contains a gap sequence (228), between the bridge sequence 3 (226), and bridge sequence 4 (224). The gap sequence (228) may optionally include the sequence of the T7 RNA polymerase promoter. The gap between the left probe and the right probe is filled by introducing a polymerase and one or more nucleotides. For this process a Stoffel fragment, Taq polymerase or Phusion polymerase can be used. The polymerase adds nucleotides (a) complimentary to the universal bridge oligo sequence and (b) complimentary to the target sequence and thereby fills in the two gaps i.e. i.e gap 1 and gap 2 between the first probe and the second probe resulting in ligation of the left probe and the right probe complementary to the bridge oligo. In such way, a barcode, if present in the bridge oligo in location 228, is integrated in the complementary sequence. Optionally, the bridge oligo probe 224 extends from the 5' site or the 3' site complimentary to the ligated probes such that a barcode, if present in probe 1, or probe 2, and/or the target sequences, 208 and 214, are integrated into the bridge oligo, thereby forming one or more ligated ligation complexes. Care should be taken, however, that the polymerase action does not interfere with the ligation of the first probe and the second probe at the site of the target sequence. It is, for instance, possible to use a polymerase that stops its action when it arrives at a double stranded DNA portion, as for instance present at the portion of the first probe and the second probe that are hybridized to the target sequence. T7 RNA polymerase promoter sequences can be embedded into the ligation complex into positions 226, 228 or 224 of the bridge oligo. The deoxyuridine moiety can be embedded between positions 204 and 206, or within 206 in the left probe.

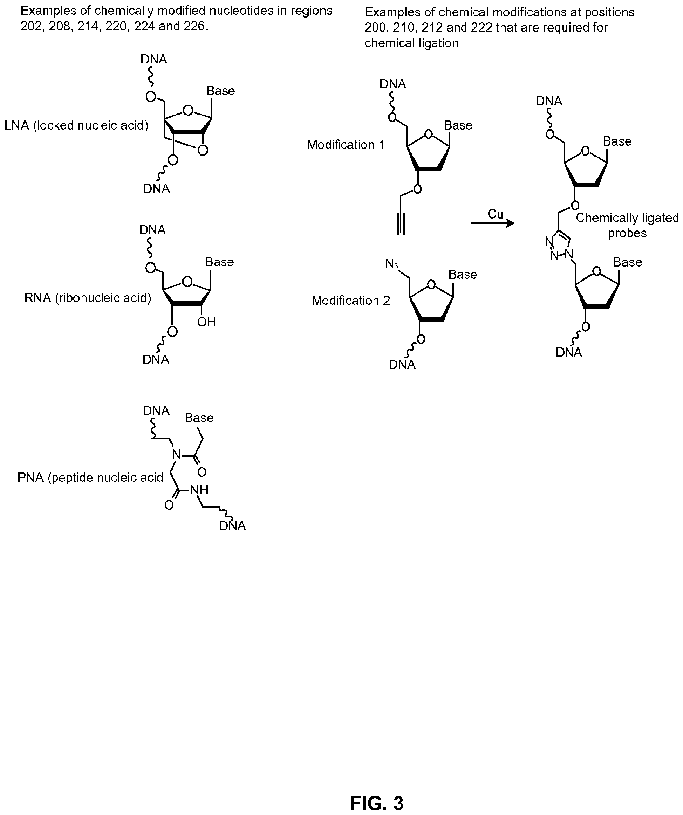

FIG. 3 is a schematic illustration of probe modification examples that improve binding to target or permit chemical ligation. The chemical modifications that improve target binding include, but are not limited to, ribonucleic acids, peptide nucleic acids and/or locked nucleic acids. The chemical modifications that permit chemical ligation include, but are not limited to, a 5'-azide 3'-alkyne modifications that can be joined in a copper-catalyzed cycloaddition.

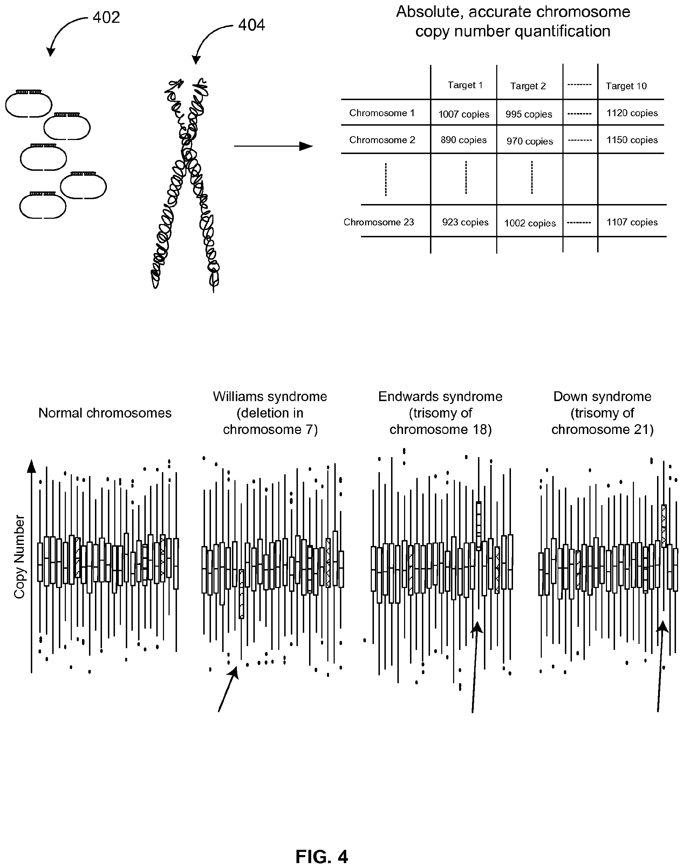

FIG. 4 provides an overview of analysis of replicated chromosomal count data using statistical tools in order to detect significant deviations from the standard deviation pattern of the chromosomal targets. The profiling of multiple genetic targets (404) in a plurality of samples is carried out using multiple target specific ligation complex mix (402). Further, the multiples genetic targets 404 include nucleic acid extracted from the maternal blood. Multiple probes target each relevant gene that can detect typical mutations. Typically, at least 10, preferably at least 100, more preferably at least 500, most preferably at least 1000 distinct probes target each chromosome for the detection of fetal chromosomal disorders from maternal blood. The use of at least 10 distinct probes results in at least 10 independent replicates for each chromosome. The set of multiple probes detect specific mutations. The replicated chromosomal count data is analyzed using statistical tools to detect significant deviations from the standard deviation pattern of the chromosomal targets. The deviations from the standard deviation pattern of the chromosomal targets permits identification of deletions such as Williams syndrome and trisomies such as Patau, Edwards or Down syndrome.

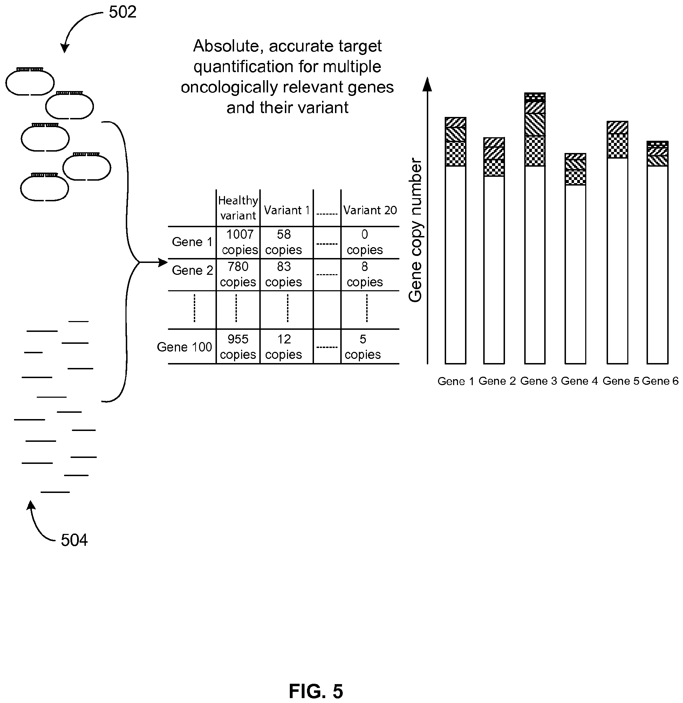

FIG. 5 provides an overview for quantitatively identifying the proportion of mutated variants of multiple genes by mixing a set of target-specific ligation complex mix (502) with multiple genetic targets (504). The multiples genetic targets (504) include nucleic acid (DNA) extracted from the blood plasma of a patient. The profiling of multiple genetic targets (504) in a very large number of samples is carried out using multiple target specific ligation complex mix (502). Each relevant gene is targeted by multiple distinct probes that can detect typical mutations. For the profiling of multiple genetic targets (504), 20 distinct probes target each gene to detect oncologically relevant mutations in circulating DNA patient suffering from cancer.

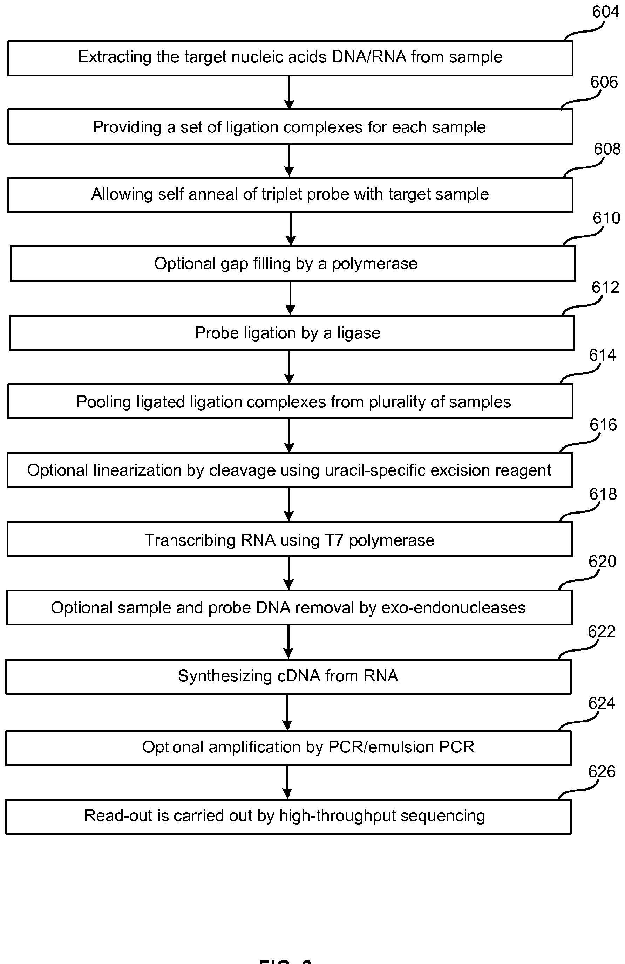

FIG. 6 illustrates a flowchart of multiplex ligation dependent probe amplification according to an embodiment herein. In step 604, (target) nucleic acid DNA/RNA is extracted from two or more samples. Such samples may be previously obtained from a human body, including biopsies, saliva and other secretions, exhaled moisture extracts, tissue, blood plasma (liquid biopsies), two or more samples obtained from environment, including water, wastewater, soil, or the like. In step 606, ligation complexes provided for each of the sample. The ligation complexes include a left probe, a right probe and a bridge oligo probe. In step 608, the target sample is mixed with the ligation complexes to allow self-annealing. In optional step 610, optionally, the gaps are filled between the first probe and the second probe and further the bridge oligo probe extension is carried out using polymerase enzyme such that circular ligated ligation complex is formed. This can, for instance, happen when the probe triplet is assembled or when the bound target-specific parts are extended and ligated. In step 612 the ligation complexes are ligated using ligase enzyme such that circular ligated ligation complex is formed. In step 614, the plurality of such ligated ligation complexes are pooled from the plurality of sample sets. In optional step 616, the optional uracil specific excision reagent is added to promote linearization by cleavage of circular ligated ligaton complex. In step 618, the T7 RNA polymerase is added that binds to the T7 RNA polymerase promoter site on the bridge oligo sequence (or on the first or second probe) of the ligated ligation complex to transcribe the RNA. In optional step 620, the DNA specific exonucleases and endonucleases are added to the reaction mixture to remove free sample and probes. In step 622, the cDNA is synthesized from RNA by reverse transcription process. In an optional step 624, the cDNA amplified by PCR or emulsion PCR. In step 626, the sequencing is carried out by means high-throughput sequencing.

## Claims

1. A method for the high-throughput detection of one or more target nucleotide sequence in a plurality of samples, the method comprising the steps of:
(i) providing for each target nucleotide sequence in each of the samples:
a first probe, a second probe and a bridge oligo,
wherein the first probe comprises, starting from the 5' end of the molecule, a first bridge oligo-specific sequence, optionally a first sequence barcode, and a first target specific portion at the 3'end of first probe;
and wherein the second probe comprises, starting from the 5' end of the molecule, a second target specific portion, optionally a second sequence barcode, and a second bridge oligo-specific sequence at the 3' end of second probe;
and wherein the bridge oligo contains sequences complementary to the first bridge oligo specific sequence and the second bridge oligo-specific sequence in the first probe and the second probe, respectively, and optionally a third barcode;
wherein at least one of the first sequence barcode or the second sequence barcode or the third barcode is present in the first probe or the second probe or the bridge oligo, respectively; and
wherein a promotor sequence for a T7 RNA polymerase is present in the first probe, the second probe or the bridge oligo,
(ii) contacting, for each of the one or more target nucleotide sequence, the first probe and the second probe with, preferably for each of the samples in a separate tube, the bridge oligo and allow self-annealing into a plurality of ligation complexes;
(iii) contacting nucleic acids present in the plurality of samples to be tested for the target nucleotide sequences with the plurality of ligation complexes;
(iv) allowing the first target specific portion and the second target specific portion of the respective first probe and the second probe to hybridize to essentially adjacent sections on the target sequence, thereby forming a hybridization complex;
(v) ligating the first probe and the second probe in the hybridization complexes to provide ligated ligation complexes,
(vi) pooling the ligated ligation complexes from the plurality of samples,
(viii) amplifying RNA from the one or more ligated complexes using T7 RNA polymerase that initiates RNA synthesis from the T7 RNA polymerase promoter embedded in the ligated ligation complexes;
(xi) optionally, preparing cDNA from the RNA molecules using a DNA-oligonucleotide molecule that is reverse-complementary to a universal site 2;
(xii) subjecting the RNA or the cDNA molecules to high-throughput sequencing technology to determine the identifier sequence(s); and
(xiii) identifying the presence and/or number of the target nucleotide sequence in the plurality of samples by determination of at least part of the first target specific portion and/or the second target specific portion, and/or at least part of the first barcode and/or the second barcode, and/or at least part of the third barcode.

2. Method according to claim 1, wherein the first probe, the second probe, or the bridge oligo comprises a deoxyuridine moiety that permits linearization by cleavage using uracil-specific excision reagent.

3. Method according to any one of claim 1-2, wherein the sequencing is carried out by means of next-generation DNA or RNA sequencing.

4. Method according to any one of claims 1-3, wherein the 3' end of the first probe or the 5' end of the second probe, or both, are modified to permit chemical ligation of the first probe to the second probe.

5. Method according to any one of claims 1-4, wherein the bridging portion of the first probe or the second probe, or both, comprise(s) chemically modified bases to permit improved binding to the bridge oligo.

6. Method according to any one of claims 1 - 5, wherein the first target specific portion, the second target specific portion, the first bridge oligo-specific sequences, and/or the second bridge oligo-specific sequences, contain independently from one another, one or more chemically modified nucleotide;

7. Method according to any one of claims 1-6, wherein genetic target enumeration is permitted by counting the number of molecular barcodes per target and per sample.

8. Method according to any one of claims 1-7, wherein the RNA molecules or the cDNA molecules are amplified with a first primer and a second primer to provide an amplification product.

9. Method according to any one of claims 1-8, wherein for two or more samples or for two or more locus/allele combinations, barcode sequences are used to genotype the sample(s) for one or more sequences and/or polymorphisms, such as SNPs and/or indels.

10. Kit of parts comprising a plurality of containers, wherein at least one container comprises one or more sets of first probe and second probe, and at least one container comprises one or more bridge oligos,
wherein the first probe comprises, starting from the 5' end of the molecule, a first bridge oligo-specific sequence, optionally a first sequence barcode, and a first target specific portion at 3' end of first probe;
wherein the second probe comprises, starting from the 5' end of the molecule, a second target specific portion, optionally a second sequence barcode, and a second bridge oligo-specific sequence at 3' end of second probe;
wherein the bridge oligo comprises sequences complementary to the first and second bridge oligo-specific sequences in the first and second probe, respectively, and optionally a third barcode; and wherein at least one of the first sequence barcode or the second sequence barcode or the third barcode is present in the first probe or the second probe or the bridge oligo, respectively; and wherein a promotor sequence for a T7 RNA polymerase is present in the first probe, the second probe or the bridge oligo,

11. Kit of parts according to claim 10, wherein the 3' end of the first probes or the 5' end of the second probes, or both, are modified to permit chemical ligation of the first probes to the second probes.

12. Kit of parts according to claim 11, wherein the bridge oligo comprises one or more chemically modified nucleotides in the sequence complementary to a sequence of the first probe or in the sequence complementary to a sequence of the second probe, or both.

13. Kit of parts according to claim 11 or 12, wherein preferably the first target specific portion, the second target specific portion, the first bridge oligo-specific sequence, and/or the second oligo-specific sequence, contain independently from one another, one or more chemically modified nucleotide.

14. Use of a first probe, a second probe and a bridge oligo for high-throughput sequencing in the determination of the presence or absence and/or quantification of at least one target sequences in a plurality of samples,
wherein the first probe comprises, starting from the 5' end of the molecule, a first bridge oligo-specific sequence, optionally a first sequence barcode, and a first target specific portion at 3' end of first probe;
and wherein the second probe comprises, starting from the 5' end of the molecule, a second target specific portion, optionally a second sequence barcode, and a second bridge oligo-specific sequence at 3'end of the second probe;
and wherein the bridge oligo comprises sequences complementary to the first bridge oligo-specific sequences and the second bridge oligo-specific sequences in the first probe and the second probe, respectively, and optionally a third barcode; wherein at least one of the first sequence barcode or the second sequence barcode or the third barcode is present in the first probe or the second probe or the bridge oligo, respectively; and wherein a promotor sequence for a T7 RNA polymerase is present in the first probe, the second probe or the bridge oligo; and wherein the first probe and the second probe are ligated when the respective target specific sections of the probes are hybridized to essentially adjacent sections on the target sequence to provide ligated probes; and the ligated probes from the plurality of samples are pooled and amplified using T7 RNA polymerase; and the amplification products are subjected to high-throughput sequencing technology to determine the identifier sequence(s).

## Patentansprüche

1. Verfahren zur Hochdurchsatzerkennung einer oder mehrerer Ziel-Nucleotid-Sequenzen in einer Mehrzahl von Proben, wobei das Verfahren die folgenden Schritte umfasst:
(i) für jede Ziel-Nucleotid-Sequenz in jeder der Proben Bereitstellen von:
einer ersten Sonde, einer zweiten Sonde und einem Brückenoligo, wobei die erste Sonde, ausgehend vom 5'-Ende des Moleküls, eine erste brückenoligospezifische Sequenz, wahlweise einen ersten Sequenz-Barcode, und einen ersten zielspezifischen Abschnitt am 3'-Ende der ersten Sonde umfasst;
und wobei die zweite Sonde, ausgehend vom 5'-Ende des Moleküls, einen zweiten zielspezifischen Abschnitt, wahlweise einen zweiten Sequenz-Barcode, und eine zweite brückenoligospezifische Sequenz am 3'-Ende der zweiten Sonde umfasst;
und wobei das Brückenoligo Sequenzen, die komplementär zu der ersten brückenoligospezifischen Sequenz und der zweiten brückenoligospezifischen Sequenz in der ersten Sonde bzw. der zweiten Sonde sind, und wahlweise einen dritten Barcode enthält;
wobei mindestens einer von dem ersten Sequenz-Barcode oder dem zweiten Sequenz-Barcode oder dem dritten Barcode in der ersten Sonde bzw. der zweiten Sonde bzw. dem Brückenoligo vorhanden ist; und wobei eine Promotorsequenz für eine T7-RNA-Polymerase in der ersten Sonde, der zweiten Sonde oder dem Brückenoligo vorhanden ist,
(ii) für jede der einen oder mehreren Ziel-Nucleotid-Sequenzen Inkontaktbringen der ersten Sonde und der zweiten Sonde, vorzugsweise für jede der Proben in einem separaten Röhrchen, mit dem Brückenoligo und Ermöglichen von Selbsttempern zu einer Mehrzahl von Ligationskomplexen;
(iii) Inkontaktbringen von Nucleinsäuren, die in der Mehrzahl von Proben vorhanden sind, die auf die Ziel-Nucleotid-Sequenzen getestet werden sollen, mit der Mehrzahl von Ligationskomplexen;
(iv) Ermöglichen, dass der erste zielspezifische Abschnitt und der zweite zielspezifische Abschnitt der ersten Sonde bzw. der zweiten Sonde mit im Wesentlichen benachbarten Abschnitten an der Zielsequenz hybridisieren, wodurch ein Hybridisierungskomplex gebildet wird;
(v) Ligieren der ersten Sonde und der zweiten Sonde in den Hybridisierungskomplexen, um ligierte Ligationskomplexe bereitzustellen,
(vi) Zusammenführen der ligierten Ligationskomplexe aus der Mehrzahl von Proben,
(viii) Amplifizieren von RNA aus dem einen oder den mehreren ligierten Komplexen unter Verwendung von T7-RNA-Polymerase, die eine RNA-Synthese aus dem T7-RNA-Polymerasepromotor, in den ligierten Ligationskomplexen eingebettet, initiiert;
(xi) wahlweise Herstellen von cDNA aus den RNA-Molekülen unter Verwendung eines DNA-Oligonucleotidmoleküls, das rückwärtskomplementär zu einer Universalstelle 2 ist;
(xii) Anwenden von Hochdurchsatz-Sequenzierungstechnologie für die RNA- oder die cDNA-Moleküle, um die Kennsequenz(en) zu bestimmen; und
(xiii) Identifizieren des Vorhandenseins und/oder der Nummer der Ziel-Nucleotid-Sequenz in der Mehrzahl von Proben durch Bestimmung zumindest von einem Teil des ersten zielspezifischen Abschnitts und/oder des zweiten zielspezifischen Abschnitts und/oder zumindest einem Teil des ersten Barcodes und/oder des zweiten Barcodes und/oder zumindest einem Teil des dritten Barcodes.

2. Verfahren nach Anspruch 1, wobei die erste Sonde, die zweite Sonde oder das Brückenoligo eine Deoxyuridin-Einheit umfasst, die eine Linearisierung durch Spaltung mithilfe eines uracilspezifischen Exzisionsreagens erlaubt.

3. Verfahren nach einem von Anspruch 1-2, wobei die Sequenzierung mittels Next-Generation-DNA- oder -RNA-Sequenzierung durchgeführt wird.

4. Verfahren nach einem der Ansprüche 1-3, wobei das 3'-Ende der ersten Sonde oder das 5'-Ende der zweiten Sonde, oder beide, modifiziert werden, um eine chemische Ligation der ersten Sonde an die zweite Sonde zu ermöglichen.

5. Verfahren nach einem der Ansprüche 1-4, wobei der Verbrückungsabschnitt der ersten Sonde oder der zweiten Sonde, oder beide, chemisch modifizierte Basen umfassen, um eine verbesserte Bindung an das Brückenoligo zu ermöglichen.

6. Verfahren nach einem der Ansprüche 1-5, wobei der erste zielspezifische Abschnitt, der zweite zielspezifische Abschnitt, die ersten brückenoligospezifischen Sequenzen und/oder die zweiten brückenoligospezifischen Sequenzen unabhängig voneinander ein oder mehrere chemisch modifizierte Nukleotide enthalten;

7. Verfahren nach einem der Ansprüche 1-6, wobei Genzielauszählung durch Zählen der Anzahl molekularer Barcodes pro Ziel und pro Probe ermöglicht wird.

8. Verfahren nach einem der Ansprüche 1-7, wobei die RNA-Moleküle oder die cDNA-Moleküle mit einem ersten Primer und einem zweiten Primer amplifiziert werden, um ein Amplifikationsprodukt bereitzustellen.

9. Verfahren nach einem der Ansprüche 1-8, wobei für zwei oder mehr Proben oder für zwei oder mehr Locus/Allel-Kombinationen Barcode-Sequenzen verwendet werden, um die Probe(n) für eine oder mehrere Sequenzen und/oder Polymorphismen, wie SNPs und/oder Indele, zu genotypisieren.

10. Teilesatz, umfassend eine Mehrzahl von Behältern, wobei mindestens ein Behälter ein oder mehrere Sets von erster Sonde und zweiter Sonde umfasst und mindestens ein Behälter ein oder mehrere Brückenoligos umfasst,
wobei die erste Sonde, ausgehend vom 5'-Ende des Moleküls, eine erste brückenoligospezifische Sequenz, wahlweise einen ersten Sequenz-Barcode, und einen ersten zielspezifischen Abschnitt am 3'-Ende der ersten Sonde umfasst;
wobei die zweite Sonde, ausgehend vom 5'-Ende des Moleküls, einen zweiten zielspezifischen Abschnitt, wahlweise einen zweiten Sequenz-Barcode, und eine zweite brückenoligospezifische Sequenz am 3'-Ende der zweiten Sonde umfasst;
wobei das Brückenoligo Sequenzen, die komplementär zu der ersten und der zweiten brückenoligospezifischen Sequenz in der ersten bzw. der zweiten Sonde sind, und wahlweise einen dritten Barcode umfasst; und wobei mindestens einer von dem ersten Sequenz-Barcode oder dem zweiten Sequenz-Barcode oder dem dritten Barcode in der ersten Sonde bzw. der zweiten Sonde bzw. der Brückenoligo vorhanden ist; und wobei eine Promotorsequenz für eine T7-RNA-Polymerase in der ersten Sonde, der zweiten Sonde oder dem Brückenoligo vorhanden ist,

11. Teilesatz nach Anspruch 10, wobei das 3'-Ende der ersten Sonden oder das 5'-Ende der zweiten Sonden, oder beide, modifiziert sind, um eine chemische Ligation der ersten Sonden an die zweiten Sonden zu ermöglichen.

12. Teilesatz nach Anspruch 11, wobei das Brückenoligo ein oder mehrere chemisch modifizierte Nucleotide in der Sequenz, die komplementär zu einer Sequenz der ersten Sonde ist, oder in der Sequenz, die komplementär zu einer Sequenz der zweiten Sonde ist, oder beiden, umfasst.

13. Teilesatz nach Anspruch 11 oder 12, wobei vorzugsweise der erste zielspezifische Abschnitt, der zweite zielspezifische Abschnitt, die erste brückenoligospezifische Sequenz und/oder die zweite oligospezifische Sequenz unabhängig voneinander ein oder mehrere chemisch modifizierte Nukleotide enthalten.

14. Verwendung einer ersten Sonde, einer zweiten Sonde und eines Brückenoligo zur Hochdurchsatzsequenzierung bei der Bestimmung der Anwesenheit oder der Abwesenheit und/oder Quantifizierung von mindestens einer Zielsequenz in einer Mehrzahl von Proben,
wobei die erste Sonde, ausgehend vom 5'-Ende des Moleküls, eine erste brückenoligospezifische Sequenz, wahlweise einen ersten Sequenz-Barcode, und einen ersten zielspezifischen Abschnitt am 3'-Ende der ersten Sonde umfasst;
und wobei die zweite Sonde, ausgehend vom 5'-Ende des Moleküls, einen zweiten zielspezifischen Abschnitt, wahlweise einen zweiten Sequenz-Barcode, und eine zweite brückenoligospezifische Sequenz am 3'-Ende der zweiten Sonde umfasst;
und wobei das Brückenoligo Sequenzen, die komplementär zu den ersten brückenoligospezifischen Sequenzen und den zweiten brückenoligospezifischen Sequenzen in der ersten Sonde bzw. der zweiten Sonde sind, und wahlweise einen dritten Barcode umfasst; wobei mindestens einer von dem ersten Sequenz-Barcode oder dem zweiten Sequenz-Barcode oder dem dritten Barcode in der ersten Sonde bzw. der zweiten Sonde bzw. dem Brückenoligo vorhanden ist; und
wobei eine Promotorsequenz für eine T7-RNA-Polymerase in der ersten Sonde, der zweiten Sonde oder dem Brückenoligo vorhanden ist; und wobei die erste Sonde und die zweite Sonde ligiert werden, wenn die jeweiligen zielspezifischen Abschnitte der Sonden mit im Wesentlichen benachbarten Abschnitten an der Zielsequenz hybridisiert werden, um ligierte Sonden bereitzustellen; und die ligierten Sonden aus der Mehrzahl von Proben zusammengeführt und unter Verwendung von T7-RNA-Polymerase amplifiziert werden; und die Amplifikationsprodukte einer Hochdurchsatz-Sequenzierungstechnologie unterzogen werden, um die Kennsequenz(en) zu bestimmen.

## Revendications

1. Procédé pour la détection à haut rendement d'une ou plusieurs séquences nucléotidiques cibles dans une pluralité d'échantillons, le procédé comprenant les étapes consistant à:
(i) fournir pour chaque séquence nucléotidique cible dans chacun des échantillons:
une première sonde, une deuxième sonde et un oligo-pont,
dans lequel la première sonde comprend, en partant de l'extrémité 5' de la molécule, une première séquence spécifique d'oligo-pont, facultativement un premier code-barres de séquence, et une première partie spécifique de cible au niveau de l'extrémité 3' de la première sonde;
et dans lequel la deuxième sonde comprend, en partant de l'extrémité 5' de la molécule, une deuxième partie spécifique de cible, facultativement un deuxième code-barres de séquence, et une deuxième séquence spécifique d'oligo-pont au niveau de l'extrémité 3' de la deuxième sonde ;
et dans lequel l'oligo-pont contient des séquences complémentaires à la première séquence spécifique d'oligo-pont et à la deuxième séquence spécifique d'oligo-pont dans la première sonde et la deuxième sonde, respectivement, et facultativement un troisième code-barres;
dans lequel au moins l'un parmi le premier code-barres de séquence ou le deuxième code-barres de séquence ou le troisième code-barres est présent dans la première sonde ou la deuxième sonde ou l'oligo-pont, respectivement;
et dans lequel une séquence de promoteur pour une ARN polymérase T7 est présente dans la première sonde, la deuxième sonde ou l'oligo-pont,
(ii) mettre en contact, pour chacun parmi la ou les séquences nucléotidiques cibles, la première sonde et la deuxième sonde avec, de préférence pour chacun des échantillons dans un tube indépendant, l'oligo-pont et permettre une auto-renaturation en une pluralité de complexes de ligature;
(iii) mettre en contact des acides nucléiques présents dans la pluralité d'échantillons à tester pour les séquences nucléotidiques cibles avec la pluralité de complexes de ligature;
(iv) permettre à la première partie spécifique de cible et à la deuxième partie spécifique de cible de la première sonde et de la deuxième sonde respectives de s'hybrider à des sections sensiblement adjacentes sur la séquence cible, ce qui forme un complexe d'hybridation;
(v) ligaturer la première sonde et la deuxième sonde dans les complexes d'hybridation pour fournir des complexes de ligature ligaturés,
(vi) regrouper les complexes de ligature ligaturés provenant de la pluralité d'échantillons,
(viii) amplifier l'ARN provenant du ou des complexes ligaturés en utilisant de l'ARN polymérase T7 qui amorce une synthèse d'ARN à partir du promoteur d'ARN polymérase T7 intégré dans les complexes de ligature ligaturés;
(xi) facultativement, préparer de l'ADNc à partir des molécules d'ARN en utilisant une molécule d'ADN-oligonucléotide qui est complémentaire inverse à un site universel 2;
(xii) soumettre l'ARN ou les molécules d'ADNc à une technologie de séquençage à haut rendement pour déterminer la ou les séquence(s) d'identifiants; et
(xiii) identifier la présence et/ou le nombre des séquences nucléotidiques cibles dans la pluralité d'échantillons par détermination d'au moins une partie de la première partie spécifique de cible et/ou de la deuxième partie spécifique de cible, et/ou d'au moins une partie du premier code-barres et/ou du deuxième code-barres, et/ou d'au moins une partie du troisième code-barres.

2. Procédé selon la revendication 1, dans lequel la première sonde, la deuxième sonde, ou l'oligo-pont comprend un fragment désoxyuridine qui permet une linéarisation par clivage en utilisant un réactif d'excision spécifique de l'uracile.

3. Procédé selon l'une quelconque des revendications 1 ou 2, dans lequel le séquençage est effectué au moyen d'un séquençage d'ADN ou d'ARN de nouvelle génération.

4. Procédé selon l'une quelconque des revendications 1 à 3, dans lequel l'extrémité 3' de la première sonde ou l'extrémité 5' de la deuxième sonde, ou l'une et l'autre, sont modifiées pour permettre une ligature chimique de la première sonde à la deuxième sonde.

5. Procédé selon l'une quelconque des revendications 1 à 4, dans lequel la partie de pont de la première sonde ou de la deuxième sonde, ou l'une et l'autre, comprend(comprennent) des bases chimiquement modifiées pour permettre une liaison améliorée à l'oligo-pont.

6. Procédé selon l'une quelconque des revendications 1 à 5, dans lequel la première partie spécifique de cible, la deuxième partie spécifique de cible, les premières séquences spécifiques d'oligo-pont, et/ou les deuxièmes séquences spécifiques d'oligo-pont, contiennent indépendamment les unes des autres, un ou plusieurs nucléotides chimiquement modifiés ;

7. Procédé selon l'une quelconque des revendications 1 à 6, dans lequel un dénombrement de cibles génétiques est permis en comptant le nombre de code-barres moléculaires par cible et par échantillon.

8. Procédé selon l'une quelconque des revendications 1 à 7, dans lequel les molécules d'ARN ou les molécules d'ADNc sont amplifiées avec une première amorce et une deuxième amorce pour fournir un produit d'amplification.

9. Procédé selon l'une quelconque des revendications 1 à 8, dans lequel pour deux échantillons ou plus ou pour deux combinaisons de locus/allèle ou plus, des séquences de code-barres sont utilisées pour génotyper le(s) échantillon(s) pour un ou plusieurs séquences et/ou polymorphismes, tels que des SNP et/ou des indels.

10. Trousse de pièces comprenant une pluralité de récipients, dans lequel au moins un récipient comprend un ou plusieurs ensembles de première sonde et deuxième sonde, et au moins un récipient comprend un ou plusieurs oligo-ponts,
dans laquelle la première sonde comprend, en partant de l'extrémité 5' de la molécule, une première séquence spécifique d'oligo-pont, facultativement un premier code-barres de séquence, et une première partie spécifique de cible au niveau de l'extrémité 3' de la première sonde;
dans laquelle la deuxième sonde comprend, en partant de l'extrémité 5' de la molécule, une deuxième partie spécifique de cible, facultativement un deuxième code-barres de séquence, et une deuxième séquence spécifique d'oligo-pont au niveau de l'extrémité 3' de la deuxième sonde ; dans laquelle l'oligo-pont comprend des séquences complémentaires aux première et deuxième séquences spécifiques d'oligo-pont dans la première et la deuxième sonde, respectivement, et facultativement un troisième code-barres;
et dans laquelle au moins l'un du premier code-barres de séquence ou du deuxième code-barres de séquence ou du troisième code-barres est présent dans la première sonde ou la deuxième sonde ou l'oligo-pont, respectivement;
et dans laquelle une séquence de promoteur pour une ARN polymérase T7 est présente dans la première sonde, la deuxième sonde ou l'oligo-pont,

11. Trousse de pièces selon la revendication 10, dans laquelle l'extrémité 3' des premières sondes ou l'extrémité 5' des deuxièmes sondes, ou l'une et l'autre, sont modifiées pour permettre une ligature chimique des premières sondes aux deuxièmes sondes.

12. Trousse de pièces selon la revendication 11, dans laquelle l'oligo-pont comprend un ou plusieurs nucléotides chimiquement modifiés dans la séquence complémentaire à une séquence de la première sonde ou dans la séquence complémentaire à une séquence de la deuxième sonde, ou l'une et l'autre.

13. Trousse de pièces selon la revendication 11 ou 12, dans laquelle de préférence la première partie spécifique de cible, la deuxième partie spécifique de cible, la première séquence spécifique d'oligo-pont, et/ou la deuxième séquence spécifique d'oligo, contiennent indépendamment les unes des autres, un ou plusieurs nucléotides chimiquement modifiés.

14. Utilisation d'une première sonde, d'une deuxième sonde et d'un oligo-pont pour un séquençage à haut rendement dans la détermination de la présence ou de l'absence et/ou la quantification d'au moins une séquence cible dans une pluralité d'échantillons,
dans laquelle la première sonde comprend, en partant de l'extrémité 5' de la molécule, une première séquence spécifique d'oligo-pont, facultativement un premier code-barres de séquence, et une première partie spécifique de cible au niveau de l'extrémité 3' de la première sonde;
et dans laquelle la deuxième sonde comprend, en partant de l'extrémité 5' de la molécule, une deuxième partie spécifique de cible, facultativement un deuxième code-barres de séquence, et une deuxième séquence spécifique d'oligo-pont au niveau de l'extrémité 3' de la deuxième sonde;
et dans laquelle l'oligo-pont comprend des séquences complémentaires aux premières séquences spécifiques d'oligo-pont et aux deuxièmes séquences spécifiques d'oligo-pont dans la première sonde et la deuxième sonde, respectivement, et facultativement un troisième code-barres;
dans laquelle au moins l'un du premier code-barres de séquence ou du deuxième code-barres de séquence ou du troisième code-barres est présent dans la première sonde ou la deuxième sonde ou l'oligo-pont, respectivement; et
dans laquelle une séquence de promoteur pour une ARN polymérase T7 est présente dans la première sonde, la deuxième sonde ou l'oligo-pont; et
dans laquelle la première sonde et la deuxième sonde sont ligaturées lorsque les sections de cible respectives des sondes sont hybridées à des sections sensiblement adjacentes sur la séquence cible pour fournir des sondes ligaturées ; et les sondes ligaturées provenant de la pluralité d'échantillons sont regroupées et amplifiées en utilisant de l'ARN polymérase T7; et
les produits d'amplification sont soumis à une technologie de séquençage à haut rendement pour déterminer la ou les séquence(s) d'identifiants.
